# EUROPÄISCHE PATENTSCHRIFT

(11) **EP 0 859 000 B1**
(45) Veröffentlichungstag und Bekanntmachung des Hinweises auf die Patenterteilung: **22.01.2003**
(21) Anmeldenummer: 98101030.9
(22) Anmeldetag: 22.01.1998
(51) Int. Cl.: C07D 261/18, C07C 235/28, C07C 235/74, C07C 237/04, G01N 33/53, A61K 39/385, A61K 31/42, A61K 31/275

(54) **Isoxazol- und Crotonsäureamidderivate und deren Verwendung als Arzneimittel und Diagnostika**
Isoxazole amide and crotonamid derivatives and drug and diagnostic uses thereof
Dérivés d'isoxazole amide et d'amide crotonique, leur utilisation comme médicament et en diagnostic

(30) Priorität: 28.01.1997 DE 19702988
(43) Veröffentlichungstag der Anmeldung: 19.08.1998
(73) Patentinhaber: Aventis Pharma Deutschland GmbH, 65929 Frankfurt am Main (DE)
(72) Erfinder: Müllner, Stefan, Dr., 65239 Hochheim (DE); Kirschbaum, Bernd, Dr., 55122 Mainz (DE); Schwab, Wilfried, Dr., 65193 Wiesbaden (DE)

(56) Entgegenhaltungen:
- EP-A- 0 013 376
- EP-A- 0 217 206
- EP-A- 0 257 882
- EP-A- 0 538 783
- EP-A- 0 607 776
- WO-A-91/17748
- WO-A-94/24095
- WO-A-96/32965
- CHEMICAL ABSTRACTS, vol. 123, no. 19, 6.November 1995 Columbus, Ohio, US; abstract no. 246331g, XP002065248 & INFLAMMATION RES., Bd. 44 (Suppl.2), 1995, Seiten S187-S188,
- CHEMICAL ABSTRACTS, vol. 122, no. 5, 30.Januar 1995 Columbus, Ohio, US; abstract no. 46004v, XP002065249 & S.M. ROBERTSON ET AL.: AGENTS ACTIONS, Bd. 42, Nr. 3/4, 1994, Seiten 167-172,
- CHEMICAL ABSTRACTS, vol. 124, no. 19, 6.Mai 1996 Columbus, Ohio, US; abstract no. 250195d, XP002065250 & K. THOSS ET AL.: INFLAMMATION RES., Bd. 45, Nr. 2, 1996, Seiten 103-107,
- CHEMICAL ABSTRACTS, vol. 122, no. 15, 10.April 1995 Columbus, Ohio, US; abstract no. 177553w, XP002065251 & V.C. DIAS ET AL.: THER. DRUG MONIT., Bd. 17, Nr. 1, 1995, Seiten 84-88,

## Beschreibung

Die Erfindung betrifft neue Isoxazol- und Crotonsäureamidderivate, ihre Herstellung und Verwendung als Arzneimittel, sowie ihre Verwendung als Antigen zur Herstellung von Antikörpern und ihre Anwendung in Diagnose- und Reinigungsverfahren.

Isoxazol- und Crotonsäureamidderivate mit entzündungshemmender, immunsuppressiver oder antiproliferativer Wirkung sind bekannt (EP 0 013 376; EP 0 217 206; EP 0 527 736). Die WO 94/24095 beschreibt Isoxazolderivate und ringoffene Isoxazolderivate mit immunmodulatorischen Eigenschaften. Darin werden auch Verfahren zur Herstellung der genannten Verbindungen beschrieben, und deren Verwendung als Arzneimittel.
Die analytische Bestimmung dieser Verbindungen in Tier- und Humanseren ist mit Hilfe herkömmlich chromatographischer Verfahren möglich. Nachteil dieser chromatographischen Verfahren ist ein hoher apparativer Aufwand, aufwendige Probenvorbereitungsschritte und geringer Probendurchsatz.

Immunologische Bestimmungs- und Analyseverfahren stellen eine schnelle und zuverlässige Alternative zu chromatographischen Verfahren dar. Entscheidend für die Durchführung dieser alternativen Verfahren ist die Gewinnung geeigneter Antikörper.

Die Erfindung bezweckt, durch Modifizierung von Isoxazol- und Crotonsäureamidderivaten Verbindungen zur Verfügung zu stellen, die sich zur Antikörpergewinnung eignen, sich an Polymere koppeln lassen und als Tracer in Radioimmunoassays einsetzbar sind.

Es wurde gefunden, daß sich Verbindungen der Formel I zur Lösung dieser Aufgabe eignen, wobei am aromatischen Ring des Anilinteils sich eine oder mehrere funktionelle Gruppen befinden, die sich als solche oder über eine Spacerfunktion kovalent an Polymere koppeln lassen.

Die Erfindung betrifft daher Verbindungen der Formel 1 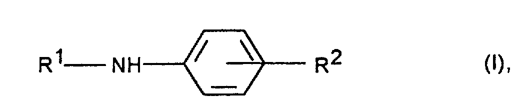 und/oder ein physiologisch verträgliches Salz der Verbindung der Formel I und/oder eine gegebenenfalls stereoisomere Form der Verbindung der Formel I,
wobei
- R¹: für den Rest der Formel II oder III steht, 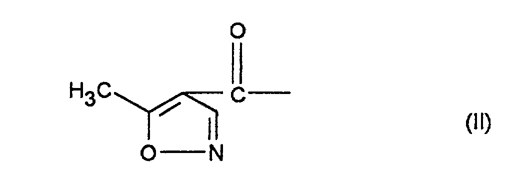 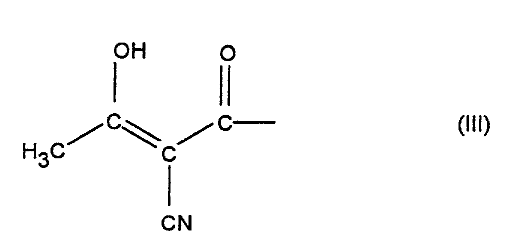
und
- R²: für
a) -O-(CH₂)ₙ-CH=CH₂, worin n die ganze Zahl 1, 2 oder 3 bedeutet, oder
b) den Rest der Formel V 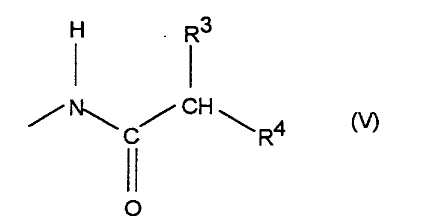 steht, worin
   R³
   1) Halogen oder
   2) -NH₂ bedeutet und

   R⁴
   1) Wasserstoffatom oder
   2) Rest einer Aminosäure bedeutet, steht.

Bevorzugt sind Verbindungen der Formel I, die dadurch gekennzeichnet sind, daß
- R¹: für den Rest der Formel II oder III steht und
- R²: für
a) -O-CH₂-CH=CH₂ oder
b) -NH-C(O)-CH(R³)(R⁴) steht,
   worin
   R³ Brom, -NH₂ oder Chlor bedeutet und R⁴ Wasserstoffatom bedeutet.

Besonders bevorzugt sind die Verbindungen der Formel I wie 2-Cyano-3-hydroxy-but-2-en-carbonsäure-(4-allyloxy-phenyl)-amid, 2-Cyano-3-hydroxy-but-2-en-carbonsäure-(4-(2-amino-acetylamino)-phenyl)-amid, 5-Methyl-isoxazol-4-carbonsäure-(4-(2-amino-acetylamino)-phenyl)-amid, 2-Cyano-3-hydroxy-but-2-en-carbonsäure-(4-(2-bromacetylamino)-phenyl)-amid oder 5-Methyl-isoxazol-4-carbonsäure-(4-(2-bromacetylamino)-phenyl)-amid

Der Rest R² in Formel I steht bevorzugt am Phenylring in para-Stellung zur "NH"-Gruppe.

Die Verbindungen der Formel I können gegebenenfalls als optische Isomere, Diastereomere, Racemate oder als Gemische derselben vorliegen. Unter dem Begriff "Aminosäure" werden die stereoisomeren Formen, z.B. D- und L-Formen, folgender Verbindungen verstanden:
Asparagin, Valin, Arginin, Asparaginsäure, Glutamin, Glutaminsäure, Tryptophan, β-Alanin, Lysin, Prolin, Glyzin, γ-Aminobutyrat, Nε-Acetyllysin, Nδ-Acetylornithin, Nγ-Acetyldiaminobutyrat, Nα-Acetyldiaminobutyrat, Histidin, Isoleucin, Leucin, Methionin, Phenylalanin, Serin, Cystein, Threonin, Alanin und Tyrosin. L-Aminosäuren sind bevorzugt. Der Aminosäurerest Gly ist insbesondere bevorzugt.

Aminosäurereste leiten sich von den entsprechenden Aminosäuren ab. Die Kurzschreibweise der Aminosäuren erfolgt nach der allgemein üblichen Schreibweise. Der Rest (R⁴) stellt die Seitenkette der jeweiligen Aminosäure dar.

Geeignete physiologisch verträgliche Salze der Verbindung der Formel I sind beispielsweise Alkali-, Erdalkali- und Ammoniumsalze einschließlich solcher von organischen Ammoniumbasen und Salze der protonierten Aminosäurereste.

Die Erfindung betrifft auch ein Verfahren zur Herstellung der Verbindung der Formel I und/oder ein physiologisch verträgliches Salz der Verbindung der Formel I und/oder eine gegebenenfalls stereoisomere Form der Verbindung der Formel I, das dadurch gekennzeichnet ist, daß man
a) eine Verbindung der Formel VI, 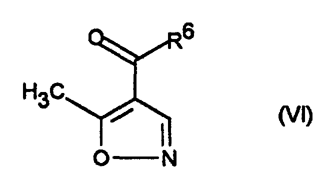 wobei R⁶ für den Rest OH, Cl oder Br steht mit einer Verbindung der Formel VII 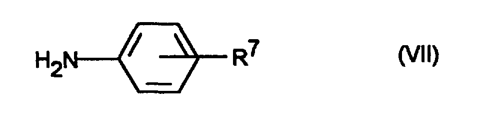 wobei R⁷ für
   1) -NH₂,
   2) -NH-C(O)-CH₂-NH-Schutzgruppe, worin Schutzgruppe eine Amin-Schutzgruppe beispielsweise Boc, darstellt,
   3) -NH-C(O)-CH₂-Halogen oder
   4) -OH
   zu einer Verbindung der Formel I umsetzt, worin R¹ den Rest der Formel II und R² -NH₂, -NH-C(O)-CH₂-NH-Schutzgruppe, -OH oder -NH-C(O)-CH₂-Halogen darstellt, und anschließend
b) eine nach Verfahren a) hergestellte Verbindung, worin R⁷ -OH bedeutet, mit einem ungesättigten Alkylhalogenid, worin der Alkylteil 3, 4 oder 5 Kohlenstoffatome hat, zu einer entsprechenden Verbindung der Formel I umsetzt, oder
c) eine nach Verfahren a) hergestellt Verbindung, worin R⁷ -NH₂ bedeutet, mit einem Carbonsäurehalogenid wie Bromacetylbromid zu einer Verbindung der Formel I umsetzt, worin R² den Rest der Formel V darstellt, R³ Halogen und R⁴ Wasserstoffatom bedeuten, oder
d) ein aromatisches Diamin wie p-Phenylendiamin mit einer an der Aminogruppe geschützten Aminosäure zu einer Verbindung der Formel VII umsetzt, worin R⁷ einen Rest der Formel V darstellt, R³ -NH₂ und R⁴ geschützte Aminosäure bedeuten und anschließend wie im Verfahren a) zu einer entsprechenden Verbindung der Formel I umsetzt, oder
e) die Schutzgruppe in einer nach Verfahren a) oder d) hergestellten Verbindung der Formel I entfernt, oder
f) eine nach Verfahren a) -e) hergestellte Verbindung der Formel I, wobei R¹ für den Rest der Formel II steht, in eine Verbindung der Formel I überführt, wobei R¹ für den Rest der Formel III, steht, oder
g) die nach Verfahren a) - f) hergestellte Verbindung der Formel I entweder in freier Form isoliert oder im Falle des Vorliegens von sauren oder basischen Gruppen gegebenenfalls in physiologisch verträgliche Salze umwandelt.

Beim Verfahrensschritt gemäß a) kann man beispielsweise eine Isoxazol-4-carbonsäure (R⁶ bedeutet -OH) nach literaturbekannten Verfahren, z.B. mittels Thionylchlorid oder Phosphoroxychlorid in einem aprotischen Lösungsmittel (z.B. Toluol, Tetrahydrofuran (THF) oder einem chlorierten Kohlenwasserstoff) in ein Säurechlorid überführen und danach mit einem aromatischen gegebenfalls in geeigneter Weise substituierten Amin unter Zusatz einer organischen Base, z.B. mit einem tertiären Amin (z.B. Triethylamin oder N-Ethylmorpholin), in einem dipolaren aprotischen Lösungsmittel, beispielsweise THF oder einem chlorierten Kohlenwasserstoff, umsetzten. Alternativ kann die Amidbildung direkt aus der Carbonsäure unter Zusatz eines aus der Peptidchemie bekannten Kondensationsreagenzes, z.B. Dicyclohexylcarbodiimid, erfolgen. Die zweite Variante eignet sich besonders für aromatische Amine mit weiteren Amino- oder Alkoholresten.

Bei dem Verfahrensschritt c) wird die über den Anilinteil eingeführte Hydroxygruppe weiter funktionalisiert, indem man dieselbe
1) mit einem Alkylhalogenid oder einem Dihalogenalkan wie 1,3-Dijodpropan unter Zusatz einer organischen oder anorganischen Base, z.B. Kaliumcarbonat, zu einem entsprechend substituierten araliphatischen Ether umsetzt, wobei man im letzteren Fall durch einen entsprechend großen Überschuß an Alkylierungsmittel oder Verwendung desselben als Solvens eine Dimerisierung vermeiden kann, so daß im Produkt eine Alkylhalogenidfunktion zur weiteren Kopplung des Produktes an eine Stationärphase verbleibt, oder
2) mit einem ungesättigten Alkylhalogenid, z.B. einem Allyl- oder Propargylhalogenid, vorzugsweise Allylbromid, in einem dipolaren aprotischen Lösungsmittel, wie Tetrahydrofuran (THF), Aceton oder chlorierten Kohlenwasserstoffen unter ähnlichen Bedingungen zu einem Allyl- oder Propargylether umsetzt, wobei unter Verwendung von Aceton als Lösungsmittel und Kaliumcarbonat als Base gleichzeitig die unter f) beschriebene Ringöffnung des Isoxazolteiles (Rest der Formel II) auftritt.

Bei dem Verfahrensschritt c) wird die Aminogruppe mit einem Carbonsäurehalogenid, z.B. Bromacetylbromid, unter Zusatz einer organischen Base wie eines tertiären aromatischen Amines (z.B. Triethylamin oder N-Ethylmorpholin ) in einem dipolaren aprotischen Lösungsmittel wie THF oder einem chlorierten Kohlenwasserstoff umgesetzt.

Bei dem Verfahrensschritt d) wird ein aromatisches Diamin, z.B. p-Phenylendiamin, mit einer an der Aminogruppe geschützten Aminosäure, z.B. N-Boc-Glycin, unter Einsatz eines Kondensationsmittels, z.B. Dicyclohexylcarbodiimid, gezielt in das Monoamid überführt, dann die Anilidbildung wie unter Verfahrensschritt a) beschrieben durchführt, und zuletzt die mitgeführte Amin-Schutzgruppe unter aus der Peptidchemie bekannten Standardbedingungen entfernt (Verfahrensschritt e)).

Bei Verfahrensschritt f) wird eine unter a) - e) hergestellte Verbindung einer baseninduzierten Ringöffnung des Isoxazolteiles unterworfen, wobei bevorzugt in einem wäßrigen-organischen Lösungsmittelgemisch, z.B. THF-Wasser oder Ethanol-Wasser, unter Verwendung eines Überschusses einer organischen oder anorganischen Base, z.B. NaOH, Ammoniaklösung oder Kaliumcarbonat, gearbeitet wird.

Sofern die Verbindungen der allgemeinen Formel I in diastereoisomeren oder enantiomeren Formen auftreten und bei der gewählten Synthese als deren Gemische anfallen, gelingt die Trennung in die reinen Stereoisomeren entweder durch Chromatographie an einem gegebenenfalls chiralen Trägermaterial, oder, sofern die racemischen Verbindungen der Formel I zur Salzbildung befähigt sind, durch fraktionierte Kristallisation der mit einer optisch aktiven Base oder Säure als Hilfsstoff gebildeten diastereomeren Salze. Auf prinzipiell gleiche Weise kann man die racemischen Verbindungen der Formel I, die eine basische Gruppe wie eine Aminogruppe enthalten, mit optisch aktiven Säuren wie (+)-Campher-10-sulfonsäure, D- und L- Weinsäure, D-und L- Milchsäure oder (+) und (-)-Mandelsäure in die reinen Enantiomeren überführen.

Die Erfindung betrifft auch Arzneimittel, gekennzeichnet durch einen wirksamen Gehalt an mindestens einer Verbindung der Formel I und/oder eine stereoisomere Form der Verbindung der Formel I und/oder ein physiologisch verträgliches Salz der Verbindung der Formel I, neben pharmazeutisch geeigneten und physiologisch verträglichen Trägerstoffen, Zusatzstoffen und/oder Wirk- und Hilfsstoffen. Die erfindungsgemäßen Arzneimittel können intravenös, parenteral, topisch, rektal oder oral verabreicht werden.

Die erfindungsgemäßen Arzneimittel eignen sich vorzugsweise zur Prophylaxe und/oder Therapie von Krebserkrankungen, Entzündungen und Autoimmunerkrankungen.

Dazu gehören beispielsweise rheumatische Erkrankungen, akute und chronische Entzündungen von Muskeln, Gelenken oder des Magen-Darm-Traktes, allergische Atemwegserkrankungen, Psoriasis oder Autoimmunerkrankungen, z.B. systemischer Lupus erythematodes (SLE), Typ-II Diabetes, Myasthenia gravis, Sjögren-Syndrom, Dermatomyositis, Sklerodermie oder Multiple Sklerose (MS). Zu den Krebserkrankungen gehören beispielsweise Lungenkrebs, Leukämie, Kaposi's Sarkom, Eierstockkrebs, Sarkome, Meningiom, Darmkrebs, Lymphknotenkrebs, Hirntumore, Brustkrebs, Magenkrebs, Pankreaskrebs, Prostatakrebs oder Hautkrebs.

Die Erfindung betrifft auch ein Verfahren zur Herstellung eines erfindungsgemäßen Arzneimittels, das dadurch gekennzeichnet ist, daß man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder flüssige galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als häufig verwendete Hilfsstoffe seien z.B. Talkum, Stärke, Magnesiumcarbonat, Titandioxid, Laktose, Mannit und andere Zucker, Milcheiweiß, Gelatine, Cellulose und ihre Derivate, tierische und pflanzliche Öle wie Lebertran, Sonnenblumen-, Erdnuß- oder Sesamöl, Polyethylenglykole und Lösungsmittel, wie etwa steriles Wasser und ein- oder mehrwertige Alkohole, z.B. Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen. Für die Behandlung eines erwachsenen, etwa 70 kg schweren Patienten sind- je nach Wirksamkeit der Verbindung der Formel I - bei Mensch und Tier Tagesdosen von etwa 50 bis 3000 mg Wirkstoff, vorzugsweise etwa 150 bis 1000 mg, bei oraler Verabreichung und von etwa 50 bis 1000 mg, bevorzugt etwa 100 bis 300 mg, bei intravenöser Applikation indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Die Erfindung betrifft ferner die Verbindung der Formel IV, die gegebenenfalls über Brückenglieder an Polymere oder Festkörper gekoppelt ist.

Dies sind Verbindungen der Formel IV 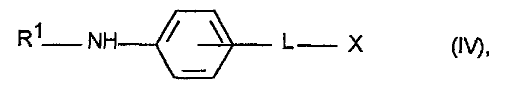 wobei
- R¹: für den Rest der Formel II oder III steht, 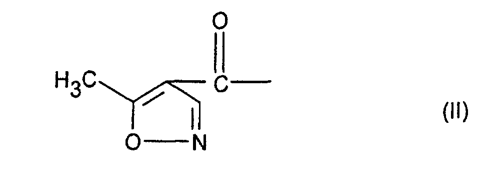 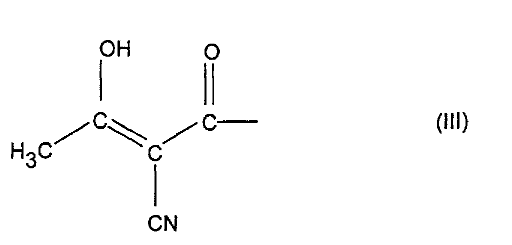
- L: für ein Brückenglied aus der Gruppe
a) -NH-C(O)-CH(R⁴)(R³),
   worin
   R³
   a) kovalente Bindung oder
   b) -NH- bedeutet und

   R⁴
   a) Wasserstoffatom oder
   b) Rest einer Aminosäure bedeutet, oder
b) -NH-(CH₂)ₘ-S-O-, worin m die ganze Zahl von 1 bis 12 ist,
c) -NH-(CH₂)ₘ-S-NR⁵-, worin R⁵ Wasserstoffatom bedeutet und m wie oben definiert ist,
d) -NH-(CH₂)ₘ-S-O-(CH₂)ₙ)-CH₂-, worin n die ganze Zahl 1, 2 oder 3 bedeutet und m wie oben definiert ist, oder
e) -NH-(CH₂)ₘ-S-NH-C(O)-CH(R⁴)(R³), wobei R³ und R⁴ wie unter a) definiert sind und m wie oben definiert ist, bedeutet, und
- X: für synthetische oder natürliche Polymere aus der Gruppe Polystyrol, Polypropylen, Polyvinylchlorid, Latex, Polysaccharide, Sepharose, Proteine, Lipide, Silikate oder Nukleinsäuren steht oder für einen Festkörper steht, der ein Röhrchen, Kugel oder Mikrotiterplatte ist.

Der Rest "L-X" in der Formel IV kann am Phenylring in meta-, ortho- oder para-Stellung zur "NH"-Gruppe stehen, bevorzugt in para-Stellung.

Unter dem Begriff "Polymer" werden synthetische oder natürliche Polymere aus der Gruppe Polystyrol, Polypropylen, Polyvinylchlorid, Latex, Polysaccharide, Sepharose, Proteine, Lipide, Silikate oder Nukleinsäuren verstanden. Die Polymere müssen gegebenenfalls mit einer oder mehreren funktionellen Resten aus der Gruppe, -OH, -COOH, -NH₂ und -CO- versehen werden, damit sie mit den Verbindungen der Formel I gekoppelt werden können. Allgemeine Methoden zur Kopplung der Verbindung der Formel I an Festkörper werden beispielsweise in BI Aapplication Handbook, Ed. 1994, Figur 4.1, (Merk AB, Uppsala, Schweden) beschrieben. Der Begriff "Festkörper" steht für unlösliche Körper, die partikulär sein können oder in geometrischen Ausführungsformen wie Röhrchen, Kugeln oder Mikrotiterplatten vorkommen.

Bevorzugt sind Verbindungen der Formel IV, wobei eine Verbindung der Formel I an einen Festkörper oder ein Polymer gekoppelt ist. Insbesondere bevorzugt sind Verbindungen der Formel IV, wobei eine Verbindung der Formel I wie 5-Methylisoxazol-4-carbonsäure-(4-(2-bromacetylamino)-phenyl)-amid oder 2-Cyano-3-hydroxy-but-2-en-carbonsäure-(4-(2-brom-acetylamino)-phenyl)-amid an einen Festkörper oder ein Polymer gekoppelt ist.

Ein weiterer Aspekt der vorliegendenen Erfindung betrifft die Verwendung der Verbindungen der Formel IV',
wobei
- R1: für den Rest der Formel II oder III wie definiert in Anspruch 1 steht,
- L: für ein Brückenglied aus der Gruppe
a) -O-,
b) -NR⁵-, worin R⁵ Wasserstoffatom bedeutet,
c) -O-(CH₂)ₙ)-CH₂-, worin n die ganze Zahl 1, 2 oder 3 bedeutet,
d) -NH-C(O)-CH(R⁴)(R³),
   worin
   R³
   a) covalente Bindung oder
   b) -NH- bedeutet und

   R⁴
   a) Wasserstoffatom oder
   b) Rest einer Aminosäure bedeutet, oder
e) ein Brückenglied L, definiert wie unter a) bis d), das über einen Spacer verfügt, worin der Spacer für einen Rest aus der Gruppe -NH-(CH₂)ₘ-S, worin m die ganze Zahl von 1 bis 12 ist, steht und
- X: für synthetische oder natürliche Polymere aus der Gruppe Polystyrol, Polypropylen, Polyvinylchlorid, Latex, Polysaccharide, Sepaharose, Proteine, Llipide, Sikikate oder Nukleinsäuren steht oder für einen Festkörper steht, der ein Röhrchen, Kugel oder Mikrotiterplatte ist, die sich zur Modifikation von Mikrotiterplatten oder zur Herstellung von Chromatographiematerial, insbesondere von Affinitätschromatographiematerial eignen.
Die zur Reinigung geeigneten Proteine stehen in direkter Wechselwirkung mit den an das Polymer oder Festkörper gebundenen Verbindungen der Formel I.

Insbesondere eignen sich als Festkörper bei den Verbindungen der Formel IV ein ®BIA core CM5-Chip oder eine Stationärphase für chromatographische Untersuchungen oder Trennungen, sowie Mikrotiterplatten.

### Beispiel 1

### 5-Methyl-isoxazol-4-carbonsäure-(4-(2-bromacetylamino)-phenyl)-amid

### Stufe a) 5-Methyl-isoxazol-4-carbonsäure-(4-amino-phenyl)-amid

10,1 g (0,08 Mol) 5-Methyl-isoxazol-4-carbonsäure und 8,65 g (0,08 Mol) p-Phenylendiamin werden in 300 ml Tetrahydrofuran gelöst und 18,05 g (0,088 Mol) Dicyclohexylcarbodiimid zugesetzt. Nach 5 Stunden (h) wird vom ausgefallenen Niederschlag abgesaugt, die organische Phase eingeengt und das Produkt an Kieselgel mittels Ethylacetat/Petrolether unter Zusatz von 1 % Eisessig chromatographiert und anschließend aus Ethylacetat/Petrolether kristallisiert. Ausbeute: 6,8 g an Acetat-Salz vom Schmelzpunkt 123 °C bis 128 °C.

### Stufe b) 5-Methyl-isoxazol-4-carbonsäure-(4-(2-bromacetylamino)-phenyl)-amid

2,17 g (0,01 Mol) des Produktes aus Stufe a) werden zusammen mit 1,9 g (0,016 Mol) N-Ethylmorpholin in 50 ml Tetrahydrofuran vorgelegt und im Eisbad eine Lösung von 2,4 g (0,012 Mol) Bromacetylbromid zugetropft und anschließend 5 h bei Raumtemperatur weitergerührt. Nach Zugabe von 5 ml Wasser wird mit 1 N HCI bis pH 2 angesäuert, das Produkt mit Ethylacetat extrahiert, die organische Phase mit Wasser gewaschen, getrocknet und eingeengt. Das Produkt wird aus Ethylacetat/Petrolether kristallisiert. Ausbeute: 2,0 g Schmelzpunkt 179 °C.
- ¹H-NMR: (DMSO-d₆):: 2,7 (s, 3H), 4,05 (s, 2H), 7,5-7,75 (m, 4H), 9,1 (s, 1H), 10,1 und 10,45 (jew. sb, 1H).

### Beispiel 2

### 2-Cyano-3-hydroxy-but-2-en-carbonsäure-(4-(2-brom-acetylamino)-phenyl)-amid

0,5 g (0,0015 Mol) des Produktes aus Beispiel 1 werden in 10 ml Tetrahydrofuran gelöst und unter Eiskühlung 2 ml 1N wäßriger NaOH zugesetzt. Die Reaktion wird per DC verfolgt und nach Beendigung (etwa nach 60-90 min) wird durch Ansäuem mit 2,25 ml 1N wäßriger Salzsäure und Zugabe von 100 ml Wasser das Produkt ausgefällt, filtriert, mit Wasser gewaschen, anschließend mit wenig Ethylacetat ausgerührt und unter verminderten Druck getrocknet. Ausbeute: 0,28 g Schmelzpunkt größer als 210 °C.
- ¹H-NMR: (DMSO-d₆):: 2,28 (s, 3H), 4,04 (s, 2H), 7,4-7,7 (m, 4H), 8,5-10 (sb, 1H), 10,4 (sb, 2H).

### Beispiel 3

### 5-Methyl-isoxazol-4-carbonsäure-(4-(2-amino-acetylamino)-phenyl)-amid Hydrochlorid

### Stufe a) 2-tertiär-Butoxycarbonylamino-acetylamino-p-phenylendiamin

8,65 g(0,08 Mol) p-Phenylendiamin werden zusammen mit 15,3 g (0,088 Mol) N-Boc-Glycin in 300 ml absoluten Tetrahydrofuran (THF) gelöst und 18,05 g (0,088 Mol) Dicyclohexylcarbodiimid portionsweise zugegeben. Nach 5 h wird vom ausgefallenen Niederschlag abgesaugt, eingeengt, das Produkt durch Chromatographie an Kieselgel mittels Ethylacetat/Methanol/Essigsäure gereinigt und danach aus Ethylacetat/Petrolether kristallisiert.
Ausbeute 13,5 g Schmelzpunkt 144 °C.

### Stufe b) 5-Methyl-isoxazol-4-carbonsäurechlorid

127,1 g (1,0 Mol) 5-Methyl-isoxazol-4-carbonsäure werden in 1 I Toluol vorgelegt, 129,8 g (1,1 Mol) Thionylchlorid zugetropft und danach für 6 h auf 80°C erhitzt. Man engt das Volumen unter verminderten Druck auf etwa die Hälfte ein und verwendet die Toluol-Lösung direkt für weitere Umsetzungen.

### Stufe c) 5-Methyl-isoxazol-4-carbonsäure-(4-(2-tertiärbutoxycarbonylaminoacetylamino)-phenyl)-amid

13,5 g (0,05 Mol) des Produktes von Stufe a) werden zusammen mit 7,6 ml (0,06 Mol) N-Ethylmorpholin in 100 ml THF vorgelegt und 30 ml der Lösung von Stufe b) (entspricht 0,06 Mol) bei 0°C zugetropft. Man rührt noch 5 h bei Raumtemperatur, hydrolysiert mit wäßriger Zitronensäure und extrahiert das Produkt mit Ethylacetat, wäscht mit Wasser, trocknet über Natriumsulfat und engt unter verminderten Druck ein. Ausbeute: 12 g Schmelzpunkt 139 °C.

### Stufe d) 5-Methyl-isoxazol-4-carbonsäure-(4-(2-amino-acetylamino)-phenyl)-amid Hydrochlorid

6 g (0,017 Mol) des Produktes von Stufe c) werden in 180 ml Dichlormethan gelöst und 18 ml Trifluoressigsäure zugesetzt. Man rührt 1 h bei Raumtemperatur, engt ein, und überführt das Produkt in das Hydrochlorid durch Lösen in Ethanol, Zugabe eines Überschusses an ethanolischer HCl, Einengen und Ausrühren des Rückstandes mit Ethylacetat. Ausbeute: 2,5 g Schmelzpunkt 210 °C.
- ¹H-NMR: (DMSO-d₆):: 2,7 (s, 3H), 3,7-3,9 (m, 2H), 7,6 und 7,72 (jew. 2H, AA'BB'), 8,25 (sb, 2H), 9,3, 10,2 und 10.75 (jew. s, 1H)

### Beispiel 4

### 2-Cyano-3-hydroxy-but-2-en-carbonsäure-(4-(2-amino-acetylamino)-phenyl)-amid Trifluoracetat

6 g (0,017 Mol) des Produktes von Beispiel 3, Stufe c) werden in 20 ml 1N Natronlauge/ 2 ml Ethanol gelöst und bei Raumtemperatur bis zur Vollständigkeit der Ringöffnung (etwa 3 h) gerührt. Beim Ansäuem mit wäßriger Zitronensäure fällt das Boc-geschützte Produkt in fester Form an und wird abgenutscht und getrocknet (Schmelzpunkt 189 °C, Ausbeute 5,5 g). 5 g dieses Zwischenproduktes werden in Dichlormethan mit Trifluoressigsäure analog zu Beispiel 3 d) behandelt und das Produkt als Trifluoracetat aus Ethanol mittels Ethylacetat und Petrolether kristallisiert. Ausbeute: 5,0 g Schmelzpunkt 209 °C.
- ¹H-NMR (DMSO-d₆):: 2,15 (s, 3H), 3,7-3,85 (m, 2H), 7,49 (s, 4H), 8,1 (sb, 3H), 10,3 und 11,2 (jew.sb, 1H).

### Beispiel 5

### 2-Cyano-3-hydroxy-but-2-en-carbonsäure-(4-allyloxy-phenyl)-amid

1,5 g (0,0068 Mol) 5-Methyl-isoxazol-4-carbonsäure-(4-hydroxy-phenyl)-amid werden in Aceton gelöst, 5,8 ml (0,068 Mol) Allylbromid und unter intensivem Rühren 9,3 g (0,068 Mol) fein gemahlenes Kaliumcarbonat zugesetzt, dann wird über Nacht bei Raumtemperatur gerührt. Man filtriert, das Filtrat wird eingeengt, der Rückstand wird in Wasser aufgenommen und aus Ethylacetat/Petrolether umkristallisiert. Ausbeute: 0,8 g Schmelzpunkt 162 °C bis 164 °C
- ¹H-NMR (DMSO-d₆):: 2,3 (s, 3H), 4,45-4,63 (m, 2H), 5,15-5,38 (m, 2H), 5,9-6,2 (m, 1H), 6,95 und 7,42 (jew. 2H, AA'BB'), 10,0 (sb, 1H), 12,5-14,5 (ssb, 1H).

### Pharmakologische Prüfungen

Als Wirksamkeitstest der Verbindungen der Formel I wird der in vitro Proliferationstest von Zellkulturen herangezogen.

### Beispiel 6

### Proliferation-Versuch

Clicks-/RPMI 1640 Medium (50:50) mit L-Glutamin ohne NaHCO₃ in Pulverform für 10 I (Seromed, Biochrom, Berlin, FRG), wird in 9 I Aqua bidest. gelöst, und steril in Flaschen mit je 900 ml filtriert.

### Waschmedium

900 ml Grundmedium werden mit 9,5 ml 7,5 %iger NatriumhydrogencarbonatLösung und 5 ml HEPES (N-2-Hydroxyethyl-Piperazin-N-2-Ethansulfonsäure) (Gibco, Eggenstein, FRG) abgepuffert.

### Gebrauchsmedium

900 ml Grundmedium plus 19 ml NaHCO₃-Lösung (7,5 %; 10 ml HEPES-Lösung und 10 ml L-Glutamin-Lösung (200 mM)).
Als Medium für die mitogeninduzierte Lymphozytenproliferation dient Gebrauchsmedium, das mit 1 % hitzeinaktiviertem (30 min, 56°C) fötalem Kälberserum (FCS) angereichert wird.

### Tumorzellmedium

Für die Haltung der Tumorzellen und Hybridomazellen wird Gebrauchsmedium mit 5 % FCS angesetzt.

### Kulturmedium für Zellinien

Für die Haltung der Zellinien werden 900 ml Gebrauchsmedium mit 10 % FCS, 10 ml NEA (non-essential amino acids)-Lösung (Gibco), 10 ml Natrium-Pyruvat-Lösung (100 mM, Gibco) und 5 ml 10⁻² M Mercaptoethanol gemischt.

### Gewinnung und Aufarbeitung der Milzzellen für die mitogeninduzierte Lymphozytenproliferation

Die Mäuse werden durch Zervikaldislokation getötet und die Milzen steril entnommen. Auf einem sterilen Sieb mit einer Maschenweite von 80 "mesh" werden die Milzen zerschnitten und mit dem Stempel einer Plastikspritze (10 ml) vorsichtig in eine Petrischale mit Gebrauchsmedium passiert. Zur Entfernung der Erythrozyten aus der Milzzellsuspension wird das Gemisch etwa 1 min, unter gelegentlichem Aufschütteln in hypotonischer, 0,17 M Ammoniumchloridlösung bei Raumtemperatur inkubiert. Die Erythrozyten werden dabei lysiert, während die Vitalität und Reaktivität der Lymphozyten nicht beeinflußt wird. Nach Zentrifugation (7 min/340 g) wird das Lysat verworfen, die Zellen zweimal gewaschen und dann im jeweiligen Testmedium aufgenommen.

### Mitogeninduzierte Lymphozytenproliferation

5x10⁵ aufgearbeitete Milzzellen aus weiblichen NMRI-Mäusen werden zusammen mit verschiedenen Mitogenen und Präparat (Verbindung der Formel I) in 200 µl Testmedium pro Vertiefung in Flachboden-Mikrotiterplatten pipettiert. Folgende Mitogen- und Präparatkonzentrationen werden verwendet:

| | |
|---|---|
| Concanavalin A [Serva] | 0,5 - 0,25 - 0,12 µg/ml |
| Lipopolysaccharid [Calbiochem] | 1,0 - 0,5 - 0,1 µg/ml |
| Phytohämagglutinin [Gibco] | 0,5 - 0,25 - 0,12 % Stammlösung |
| Pokeweed mitogen [Gibco] Verbindung 1 oder 2 | 50, 25, 10, 7,5, 5, 2,5, 1, 0,5, 0,1 µMol. |

Als Positivkontrollen werden die Gruppe mit Mitogenzusätzen ohne Präparat definiert. Bei den Negativkontrollen handelt es sich um Zellen in Kulturmedium mit Präparat ohne Mitogenzusätze. Jede Mitogenkonzentration wird mit allen Präparatkonzentrationen vierfach getestet. Nach 48 h Inkubation bei 37°C/5 % CO₂ wird den Zellen 25 µl/Vertiefung Tritium-Thymidin (Amersham) mit einer Aktivität von 0,25 µCi/Vertiefung (9,25x10³ Bq) hinzugefügt. Es schließt sich eine weitere Inkubation unter den gleichen Bedingungen für einen Zeitraum von 16 h an. Zur Auswertung des Testansatzes werden die Zellen über ein Zellerntegerät (Flow Laboratories) auf Filterpapier geemtet, wobei nicht eingebautes Thymidin in einer gesonderten Abfallflasche gesammelt wird. Das Filterpapier wird getrocknet, ausgestanzt und zusammen mit 2 ml Szintillator (Rotiszint 22, Firma Roth) in Szintillationsgefäße gegeben, die dann noch 2 h bei 4°C gekühlt werden. Die Menge, der von den Zellen eingebauten Radioaktivitäten, wird in einem Beta-Zähler (Firma Packard, Tricarb-460c) gemessen.

### Aufbereitung der Tumorzellen und Zellinien für den Proliferation-Test

Die im Test verwendeten Tumorzellen oder Zellinien werden in der logarithmischen Wachstumsphase der Stammhaltung entnommen, zweimal mit Waschmedium gewaschen und im entsprechenden Medium suspendiert.

### Durchführung und Auswertung der Proliferation-Tests

Der Proliferation-Test wird in Rundboden-Mikrotiterplatten durchgeführt. Verbindungen der Formel I und Interleukine werden in je 50 µl/Vertiefung des entsprechenden Mediums gelöst und die Zellzahl (5x10⁵) wird mit 100 µl/Vertiefung eingestellt, so daß sich ein Endvolumen von 200 µl/Vertiefung ergibt. In allen Tests werden die Werte vierfach bestimmt. Zellen ohne Präparate und ohne Wachstumsfaktor werden als Negativkontrolle definiert und Zellen ohne Präparat mit Wachstumsfaktor ergeben die Werte für die Positivkontrolle. Der Wert der Negativkontrolle wird von allen ermittelten Werten abgezogen und die Differenz aus Positivkontrolle minus Negativkontrolle wird als 100 % gesetzt.
Die Mikrotiterplatten werden 72 h bei 37 °C/5 % CO₂ inkubiert und die Proliferationsrate wird entsprechend wie bei der mitogeninduzierten Lymphozytenproliferation bestimmt.
Die Zellinien sind der Stammsammlung, American Type Culture Collection (ATCC), entnommen worden.

Die Tabelle 1 zeigt die Konzentrationen bei denen eine 50 %ige Hemmung auftritt:

| Verbindung der Formel I gemäß Beispiel | Zellinie | | |
|---|---|---|---|
| | A20.2.J (µM) | EL 4 (µM) | K562 (µM) |
| 1 | 0,8 | 0,8 | 50 |
| 2 | 15,0 | 6,0 | 20 |
| 5 | 50,0 | 25,0 | nicht bestimmt |

### Beispiel 7

### Verfahren zur Bindung der Verbindung gemäß Beispiel 2 an die ®BIAcore CM5-Matrix

Ausgangsmaterial ist ein CM5-Chip der Firma Pharmacia Biosensor mit Carboxymethyldextran-Oberfläche (BIAapplication Handbook, Ed. 1994, Merk AB, Uppsala, Schweden) . Die Kopplungsschritte erfolgen alle bei einer Flußrate von 5 µl/min. Alle Schritte werden bei 25°C mit Hepes gepufferte Salzlösung (HBS) pH 7,4, als Laufpuffer durchgeführt.
1. Schritt: 35 µl einer 1:1-Mischung von 0,05 M NHS (N-Hydroxysuccinimid) und 0,2 M EDC (N-Ethyl-N'-(Dimethylaminopropyl)carbodiimid), um die Chip-Oberfläche zu aktivieren.
2. Schritt: 35 µl einer 40 mM Cystamindihydrochlorid-Lösung in 0,1 M Natrium-Borat-Puffer pH 8,5.
3. Schritt: 35 µl Ethanolamin-Hydrochlorid pH 8,5 zur Absättigung nicht belegter Matrixstrukturen.
4. Schritt: 35 µl 0,1 M Dithiothreitol in 0,1 M Natrium-Borat-Puffer pH 8,5.
5. Schritt: 35 µl einer Lösung enthaltend Verbindung gemäß Beispiel 2 (10 mg/ml) in 0,1 M Natrium-Borat-Puffer pH 7,5.

### Beispiel 8

### Für die Analysen angewendeten ®BIAcore-Methode

Die Durchführung der Analyse erfolgte im wesentlichen wie in Current Biology beschrieben (Vol. 3, Nr. 12, 1993, Seiten 913-915).

| | |
|---|---|
| System | ®BIAcore 2000 mit entsprechender Software, Pharmacia Biosensor AB, Uppsala, Schweden |
| Chip | CM5 Sensorchip, Pharmacia Biosensor AB |
| Belegung | gemäß Beispiel 8 |
| Laufpuffer | HBS Puffer, BIA certified, Pharmacia Biosensor AB |
| Flußrate | 10 µl/min |
| Injektion | je 50 µl der zu analysierenden Probe 5 min lange Assoziationsphase |
| Spülzeit | 180 sec 3 min lange Dissoziationsphase |
| Regeneration | 2 x 15 sec mit 0,05 % Natriumdodecylsulfat |

### A) Bindung verschiedener Serumalbumine

| Art | Resonance Units |
|---|---|
| Rind | 50 |
| Mensch | 294 |
| Ratte | 1061 |
| Maus | 151 |
| Huhn | 8 |
| Esel | 480 |
| Schaf | 331 |

Der Begriff "Resonance Units" steht für eine Mengeneinheit die proportional zur gebundenen Proteinmenge ist.

### B) Bindung verschiedener Dehydrogenasen

| Enzym | Resonance Units |
|---|---|
| Glycerinaldehydphosphat-DH (25 nM) | 69 |
| Glycerinaldehydphosphat-DH (50 nM) | 148 |
| Glycerinaldehydphosphat-DH (100 nM) | 297 |
| Glycerinaldehydphosphat-DH (250 nM) | 603 |
| Dihydroorotat-DH (50 nM) | 203 |
| Dihydroorotat-DH (100 nM) | 425 |
| Dihydroorotat-DH (250 nM) | 1064 |
| Pyruvatkinase (50 nM) | 85 |
| Pyruvatkinase (100 nM) | 189 |
| Pyruvatkinase (250 nM) | 505 |

### DH steht für die Enzymbezeichnung Dehydrogenase

Die relativen Bindungsstärken der untersuchten Dehydrogenasen konnten bei einer definierten Proteinkonzentration miteinander verglichen werden. Bei 1 µM Proteinkonzentration ergab sich, daß die Bindung der Dihydroorotat-DH am stärksten war, gefolgt von Lactat-DH, Glycerinaldehydphosphat-DH und Pyruvatkinase.

### Beispiel 9

### Affinitätschromatographie

Die Verbindung gemäß Beispiel 1 wird über seine reaktive Bromgruppe an eine Trägermatrix gekoppelt. Als Trägermaterial wird Fractogel® EMD-SH (Firma Merck KGaA, Darmstadt) eingesetzt. Die kovalente Bindung erfolgt nach einem Standardprotokoll gemäß DE 43 10 964. Das erhaltene Gel wird in eine Superformance® (1 cm x 5 cm) Säule (Fa. Merck KGaA) gefüllt und an eine Hochdruckflüssigchromatographie-Anlage (HPLC) angeschlossen.

### Gewinnung der löslichen Zellproteine

Der Stamm RAW 264.7 (ATCC Stammsammlung) wird 48 Stunden (h) bis zum Erreichen der Konfluenz kultiviert und dreimal mit 4°C kaltem PBS-Puffer vom Kulturmedium frei gewaschen, die Zellen werden in PBS-Puffer überführt und mit 200 g für 10 min zentrifugiert. Die Zellaufarbeitung wird bei 4°C durchgeführt. Das Zellpellet wird in Puffer A, bestehend aus 20 mM Tris-Base, 2 mM MgCl₂ x 6 H₂O und 1 mM Dithiotreitol (DTT), resuspendiert. Eine Mischung verschiedener Proteaseinhibitoren wird zugesetzt, um eine Protolyse durch zelleigene Proteasen zu verhindern. Anschließend wird in einem Glaspotter homogenisiert. Die Suspension wird anschließend zur Gewinnung der löslichen Proteine, bei 4°C 30 min mit 22000 g zentrifugiert.
Der Überstand wird sofort für die Affinitätschromatographie weiterverwendet. Ein Aliquot des Überstandes wird als Referenz zurückbehalten, der Rest auf die Affinitätssäule gepumpt.

### HPLC

Für die Affinitätschromatographie wird eine Apparatur von Kontron Instruments, Milano, Italien eingesetzt. Das System besteht aus den Komponenten Autosampler 465, HPLC Pump 422 und 422S, einem Hochdruckmischventil M800, einem Besta Motorventil und einem Dioden Array Detektor 440. Die Datenaufnahme und Auswertung erfolgt mit dem Data System 450-MT2/DAD series. Der Zellüberstand wird mit Wasser auf ein Volumen von 30 ml verdünnt und mit einem Fluß von 1 ml/min aufgepumpt. Der Durchbruch wird aufgefangen und ebenso wie die Fraktionen bis zur Beendigung der Chromatographie bei 4°C gelagert.
Für die Gradientenelution der Proteine werden folgende Puffer verwendet:

| Zusammensetzung des PBS-Puffers: | |
|---|---|
| NaCI | 8,00 g/l |
| KCI | 0,20 g/l |
| KH₂PO₄ | 0,20 g/l |
| Na₂HPO₄ x 7H₂O | 2,16 g/l |

| Zusammensetzung der in Tabelle 2 verwendeten Puffer: | |
|---|---|
| Puffer: | Zusammensetzung: |
| 1 | PBS |
| 2 | PBS + 0,5 M NaCl |
| 3 | PBS + 150 mM A 77 1726b (Natriumsalz von N-(4-Trifluormethylphenyl)-2-cyan-3-hydroxycrotonsäureamid) |
| 4 | H₂O |
| 5 | 6 M Harnstoff |
| 6 | 60 % Acetonitril + 0,1 % Trifluoressigsäure (TFA) |

Nach dem Aufpumpen der Proteinlösung wird mit einem Fluß von 3 ml/min der Gradient gemäß Tabelle 2 gestartet.

**Tabelle 2**

| Zeit [min] | Gradient | Fraktion |
|---|---|---|
| 0 - 10 | H₂O (4) | 1 |
| 10 - 15 | Gradient auf PBS (1) | 2 |
| 15 - 20 | PBS (1) | |
| 20 - 35 | Gradient auf PBS + 0,5 M NaCl (2) | 3 |
| 35 - 40 | PBS + 0,5 M NaCl (2) | |
| 40 - 50 | Gradient auf PBS (2) | |
| 50 - 55 | PBS (1) | 4 |
| 55 - 70 | Gradient auf PBS + Präparat (3) | 5 |
| 70 - 75 | PBS + Präparat (3) | |
| 75 - 90 | H₂O (4) | 6 |
| 90 - 100 | Harnstoff (5) | 7 |
| 100 - 110 | H₂O (4) | 8 |
| 110 - 120 | Acetonitril (6) | 9 |

### Die Zahlen in Klammer beziehen sich auf den verwendeten Puffer

Die Fraktionen werden gesammelt, 48 h bei 4°C gegen H₂O dialysiert (Ausschlußgrenze der verwendeten Dialysemembran 6000 - 8000 Da) (außer Acetonitril-Fraktion) und anschließend lyophilisiert.

### Proteinbestimmung

Nach dem Lyophilisation werden die Proteine in 1 ml 1 % Natriumdodecylsulfat (SDS) gelöst und mit Wasser auf 0,5 % SDS verdünnt. Von dieser Lösung werden ein Aliquot entnommen und zur Proteinbestimmung mit dem BCA-Test (Pierce) eingesetzt. Die Proteine werden eingedampft (UNIVASPO 150H, UniEquip Power Heater, Martinsried, Deutschland) und anschließend in einem geeigneten Volumen Probenpuffers, bestehend aus 10 g Saccharose, 9 ml 0,25 M Tris/1 M Glyzin-Lösung, 7,8 ml 10 % SDS, 2,5 ml Bromphenolblau (0,1 %), 4 ml β-Mercaptoethanol und 1,7 ml Wasser aufgenommen.

### SDS-PAGE

Zur Herstellung eines SDS-Gels mit einem Polyacrylamidgradienten von 10 - 17 % werden folgende Lösungen angesetzt.

| A: | | B: | |
|---|---|---|---|
| 5 ml | Acrylamid (30 %, 0,5 %) | 8,5 ml | Acrylamid (30 %, 0,5 %) |
| 3,75 ml | Tris (3 M, pH 8,8) | 3,75 ml | Tris (3M, pH 8,8) |
| 6,12 ml | Wasser | 3,275 ml | Glycerin |
| 0,15 ml | SDS | 0,15 ml | SDS |

Die Polymerisation wird durch Zusatz von 100 µl 10 % APS und 12,5 µl TEMED gestartet. Das Sammelgel wird aus 3 ml Acrylamid (10 %, 0,5 %), 1,36 ml Wasser, 1,45 ml 0,5 M Tris (pH 6,8), 60 µl 10 % SDS, 106 µl 10 % APS und 9 µl TEMED hergestellt.
Für die Herstellung der analytischer Gele werden etwa 10 µg Protein pro Bahn geladen, für präparative Gele 100 µg.
Die Bestimmung der Molekulargewichte erfolgt durch Vergleich mit Protein Standard Mischungen.

### Sequenzierung

Proteinfraktionen, die im analytischen Gel Banden angereicherten Proteine zeigen, werden im präparativen Maßstab getrennt und die getrennten Proteine durch Blotten auf einer PVDF-Membran (Millipore) immobilisiert. Die Proteine werden durch Färben mit Coomassie sichtbar gemacht und dann direkt dem Edman-Abbau unterworfen. Proteine, deren N-Terminus blockiert ist, werden nach der Elektrophorese aus Coomassie gefärbten Gelen sorgfältig ausgeschnitten und mit Wasser neutral gewaschen. Die Gelstückchen werden durch zwei hintereinanderliegende Stahlsiebe mit einer Maschenweite von 100 µm und 32 µm gepreßt und so homogenisiert. Die salbenartige Masse wird in der UNIVAPO 150 H (UniEquip Power Heater, Martinsried, Deutschland) bis auf eine geringe Restfeuchte getrocknet. Die so erhaltenen Proteine werden durch 7 h Inkubation bei 37°C mit dem Enzym Endoproteinase LysdC (Boehringer Mannheim) (Enzym zu Protein Verhältnis etwa 1:10) in einem Puffer aus 25 mM Tris-HCI, pH 8,5 und 1 mM EDTA, in Peptide gespalten.
Die erhaltenen Peptide werden zweimal mit je 1 ml 60 % Acetonitril, 0,1 % TFA 4 h bei 37 °C eluiert. Die vereinigten Eluate werden in der UNIVAPO eingedampft, und die erhaltenen Peptide werden durch Reversed-Phase-Chromatographie getrennt. Die Chromatographie wird auf einer LiChroCART® 125-2 Superspher® 60 Säule mit einem linearen Gradienten (1 %/min) von A: 0,1 % TFA in H₂O auf B: 0,1 % TFA in Acetonitril in 70 min durchgeführt.
Die Aminosäuresequenz der gereinigten Peptide wird nach dem Prinzip des Edman-Abbaus an einem 477 A Gasphasen-Sequenzer (Applied Biosystems) durchgeführt. Die Identifizierung der PTH-AS erfolgt an einem 120A Phenylthiohydantoin Aminosäure Analysator (Applied Biosystems) bei 269 nm.

### Western-Blot Analyse

Nach dem Transfer von Proteinen aus einem SDS-Gel auf die PVDF-Membran (Millipore), wird die nicht belegte Fläche durch 2 h Inkubation mit 2,5 % Hühnereiweiß (Sigma) in TBS, bestehend aus 45 g NaCl, 30,3 g Tris-Base in 5 I, pH 7,4 blockiert.
Der erste Antikörper wird in einem geeigneten Verhältnis mit Blocking-Puffer verdünnt und 1 h inkubiert. Der Blot wird anschließend dreimal 5 min mit TBS und 0,1 % Tween 20 gewaschen und 1 h mit dem zweiten, POD markierten Antikörper inkubiert. Der Waschvorgang wird wiederholt und der gebundene Antikörper durch eine Lösung aus 60 mg 4-Chlor-1-Naphthol, 20 ml Methanol, 100 ml TBS und 200 µl Wasserstoffperoxid (30 %) sichtbar gemacht.

Die Proteine, die in der NaCI (3) - und A771726b (5) -Fraktion (siehe Tabelle 2) erhalten wurden, konnten anhand von sieben bis vierzehn Aminosäure-langen Peptiden identifiziert werden. Im Fall des 22kDa großen Proteins konnte die ermittelte Aminosäuresequenz zwei Proteinen, MSP23 und NKEF-A, zugeordnet werden. Die Ursache hierfür liegt in der hohen Sequenzhomologie der beiden Proteine von 93 %. Die Sequenzierungsergebnisse der Affinitätschromatographie wurden in Tabelle 3 zusammengefaßt.

**Tabelle 3:**

| Fraktion | Protein | Aminosäuresequenz | MW [Da] |
|---|---|---|---|
| NaCl(3) | Lactat-DH M-chain (LDH-A) | SADTLWGIQK | 36498 |
| | Cyclophilin A | TAENFRALSTGEK | 17971 |
| A 77 1726 B (5) | HSP90-β (HSP84) | EQVANSAFVERVRK | 83185 |
| | HSP70 (HSC73) | EIAEAYLGK | 70871 |
| | Pyruvatkinase M₂ | GPEIRTGLIK | 57755 |
| | EF1-α | STTTGHLIYK | 50164 |
| | Actin (γ-chain) | EITALAPSTMKRGILTLK | 41876 |
| | GAPDH | VIPELNGK | 35679 |
| | Malat-DH | ITPFEEKVVE-FV | 35596 |
| | Lactat-DH | NLRRVHP | 36367 |
| | Phosphoglycerat-Mutase | PMQFLGDEETVRK | 28635 |
| | MSP23 / NKEF-A | ATAVMPDGQFK | 22176 |

In Klammern steht in der Spalte Fraktion die Fraktions-Nr. gemäß Tabelle 2.

### Beschreibung der identifizierten Proteine

Das in der Fraktion (3) angereicherte 18 kDa große Protein wurde als Cyclophilin A identifiziert. Cyclophilin A gehört zur großen Familie der Peptidylpropyl-cis-trans-Isomerasen, die am Wirkmechanismus einer Reihe von immunsuppressiven Präparaten, z.B. Cyclosporin A beteiligt sind.

Zusätzlich wurde in der Fraktion (3) die Lactat-Dehydrogenase identifiziert, ein Enzym der anaeroben Glykolyse, das die Reaktion von Pyruvat zu Lactat katalysiert. Die Lactat-Dehydrogenase kommt im tierischen Gewebe in mindestens fünf verschiedenen Isoenzymformen vor. Das im Skelettmuskel vorherrschende Isoenzym enthält vier A-Ketten, das im Herz dominierende vier B-Ketten. Im vorliegenden Fall wurde ein Peptid aus der A-Kette der Lactat-Dehydrogenase identifiziert.

In der Fraktion (5) wurden zwei Proteine identifiziert, die zur Familie der Hitzeschock-Proteine gehören. Das bei Eukaryonten dominierende Hitzeschock-Protein HSP70 ist Mitglied einer Multigenfamilie.
Das Hitzeschock-Protein HSP90 ist ein cytosolisches Protein, daß konstitutiv auch unter normalen Bedingungen exprimiert wird. Es besteht aus zwei separaten Genprodukten, HSP84 und HSP86, die untereinander eine Sequenzhomologie von 86 % besitzen. Von HSP90 ist bekannt, daß es einen Komplex mit Steroidhormon-Rezeptoren bildet und auf diese Weise in die Transkription bestimmter Gene eingreift.

In der Fraktion (5) wurden mit Glycerinaldehyd-3-Phosphat Dehydrogenase (GAPDH), Pyruvatkinase M₂ und Phosphoglycerat-Mutase außerdem drei Proteine der Glykolyse gefunden. Diese drei Enzyme stammen aus dem unteren Abschnitt der Glykolyse und liegen unter nativen Bedingungen in einem Multienzymkomplex vor.

Eine Bande, die ebenfalls sehr stark in der Fraktion (5) angereichert wurde, konnte als Elongationsfaktor (EF-1α) identifiziert werden. EF-1α ist ein bedeutender eukaryontischer Translationsfaktor. Er ist in seiner Funktion vergleichbar mit dem prokaryontischen Elongationsfaktor EF-Tu und befördert während der Proteinbiosynthese in einem GTP abhängigen Prozeß Aminoacyl-tRNAs aus dem Cytosol zu ihrer Akzeptorstelle am Ribosom.

Zusätzlich konnte Actin in der Fraktion (5) nachgewiesen werden. Actin ist ein bei Eukaryonten nahezu überall und in großen Mengen vorkommendes Protein. Es ist Hauptbestandteil der Muskulatur sowie des Cytoskelettes. Die Actin Multigenfamilie kodiert für mindestens vier Muskel-Actinformen sowie für zwei cytoplasmatische Actinformen (β- und γ-Actin). Bei dem hier vorliegenden Actin handelt es sich um die γ-Kette der cytoplasmatischen Actinform.

Ferner wurde ein Enzym des Citronensäurezyklus und des Malat-Aspartat Shuttles, die Malat-Dehydrogenase, identifiziert. Die Malat-Dehydrogenase kommt im tierischen Gewebe in einer cytosolischen und einer mitochondrialen Isoform vor. Beide Isoenzyme spielen in Kooperation mit der Aspartat-Aminotransferase eine wichtige Rolle im Malat-Aspartat Shuttle zwischen Cytosol und Mitochondrium.

Das 22 kDa große Protein der Fraktion (5) konnte aufgrund des sequenzierten Peptidfragmentes zwei Proteinen zugeordnet werden: dem Makrophagen-23kDa-Stress Protein (MSP23) auch bekannt als Osteoblasten spezifischer Faktor-3 (OSF-3) und dem "Natural Killer Cell Enhancing Factor-A" (NKEF-A). Aktivierte Makrophagen produzieren reaktive Sauerstoffverbindungen wie H₂O₂ und O₂. Da sie selbst diesem oxydativen Stress widerstehen, müssen sie ein wirkungsvolles Abwehrsystem besitzen, um sich vor diesen reaktiven Verbindungen schützen zu können. Bei NKEF handelt es sich um ein cytosolisches Protein aus humanen roten Blutzellen, das die Aktivität der natürlichen Killerzellen verstärkt. Diese zählen zu den Lymphozyten und sind nach Cytokinstimulation in der Lage, eine Vielzahl von Tumorzellen zu erkennen und zu zerstören. Der "Natural Killer Cell Enhancing Factor" hat ein Molekulargewicht von 44 kDa und besteht aus zwei Untereinheiten gleicher Größe (NKEF-A und NKEF-B), die untereinander eine gute Sequenzhomologie von 88 % besitzen.

### Beispiel 10

### Vergleichende Affinitätschromatographie von unstimulierten, LPS stimulierten und LPS stimulierten, Leflunomid behandelten RAW 264.7 Cytosolextrakten.

Als in vitro Modell für entzündliche Prozesse wurde die Stimulation der Makrophagenzellinie RAW 264.7 mit Lipopolysaccharid verwendet. Lipopolysaccharid (LPS) ist ein essentieller struktureller Bestandteil der äußeren Membran Gram-negativer Bakterien und wird als solcher von Immunzellen nahezu aller Organismen erkannt. Insbesondere Makrophagen werden durch LPS-Stimulation zur Synthese einer Anzahl von Cytokinen wie TNF-α, IL-1 und IL-6 angeregt. Potentielle Veränderungen im Proteinmuster, die mit dieser Stimulation einhergehen, sollten durch die gleichzeitige Gabe des immunregulatorischen Präparates Leflunomid verhindert werden.

RAW 264.7-Zellen wurden für 24 Stunden mit 10 ng LPS/ml Kulturmedium inkubiert. Im Falle von LPS stimulierten, leflunomid-behandelten Zellen wurde gleichzeitig für 24 Stunden mit 60 µM A 77 1726B inkubiert. Die Zellen wurden aufgearbeitet und die cytosolischen Extrakte anhand des gemäß Beispiels 10 derivatisierten Fractogel®-Säule affinitätschromatographisch untersucht. Die Präparate-Fraktionen dieser drei Ansätze wurden anschließend auf ein analytisches Gradientengel aufgetragen und miteinander verglichen.

Besonders markant war die Zunahme einer Bande um 35kDa, die bereits durch Sequenzierung als Malat-Dehydrogenase identifiziert worden war. Im Vergleich zu den übrigen Proteinbanden, die tendenziell in ihrer Intensität bei Inkubation mit LPS und A 77 1726B schwächer wurden, nahm die Intensität dieser Bande deutlich zu.

## Patentansprüche

1. Verbindung der Formel I 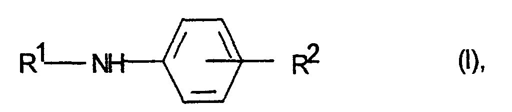 und/oder ein physiologisch verträgliches Salz der Verbindung der Formel I und/oder eine gegebenenfalls stereoisomere Form der Verbindung der Formel I, wobei
R¹ für den Rest der Formel II oder III steht, 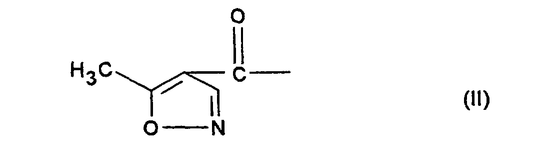 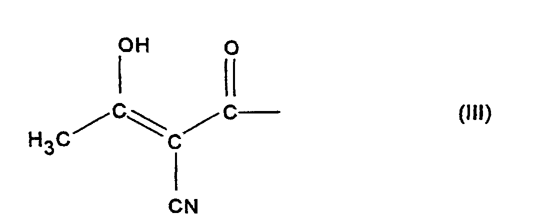
und
R² für
a) -O-(CH₂)ₙ-CH=CH₂, worin n die ganze Zahl 1, 2 oder 3 bedeutet, oder
b) den Rest der Formel V 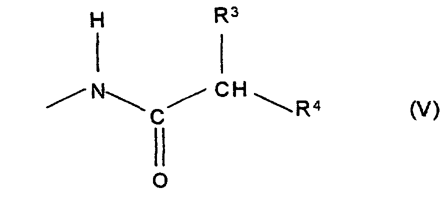 steht, worin
R³
1) Halogen oder
2) NH₂ bedeutet und
R⁴
1) Wasserstoffatom oder
2) Rest einer Aminosäure bedeutet.

2. Verbindung der Formel I gemäß Anspruch 1, **dadurch gekennzeichnet, dass**
R¹ für den Rest der Formel II oder III steht und
R² für
a) -O-CH₂-CH=CH₂ oder
b) -NH-C(O)-CH(R³)(R⁴) steht,
worin
R³ Brom, -NH₂ oder Chlor bedeutet und
R⁴ Wasserstoffatom bedeutet

3. Verbindung der Formel 1 gemäß Anspruch 1 oder 2, **dadurch gekennzeichnet, dass** der Rest R² in Formel I am Phenylring in para-Stellung zur "NH"-Gruppe steht.

4. Verbindung der Formel I gemäß Anspruch 1 wie
2-Cyano-3-hydroxy-but-2-en-carbonsäure-(4-allyloxy-phenyl)-amid,
2-Cyano-3-hydroxy-but-2-en-carbonsäure-(4-(2-amino-acetylamino)-phenyl)-amid Trifluoracetat,
5-Methyl-isoxazol-4-carbonsäure-(4-(2-amino-acetylamino)-phenyl)-amid Hydrochlorid,
2-Cyano-3-hydroxy-but-2-en-carbonsäure-(4-(2-brom-acetylamino)-phenyl)-amid oder
5-Methyl-isoxazol-4-carbonsäure-(4-(2-bromacetylamino)-phenyl)-amid

5. Verfahren zur Herstellung der Verbindung der Formel I gemäß einem oder mehreren der Ansprüche 1 bis 4, **dadurch gekennzeichnet, dass** man
a) eine Verbindung der Formel VI, 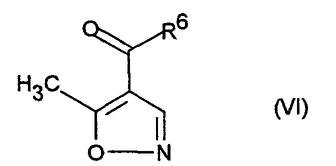 wobei R⁶ für den Rest OH, Cl oder Br steht mit einer Verbindung der Formel VII 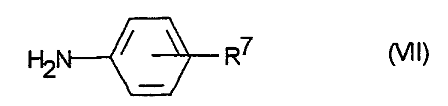 wobei R⁷ für
1) -NH₂,
2) -NH-C(O)-CH₂-NH-Schutzgruppe,
worin Schutzgruppe eine Amin-Schutzgruppe, insbesondere Boc, darstellt,
3) -NH-C(O)-CH₂-Halogen oder
4) -OH
zu einer Verbindung der Formel I umsetzt, worin R¹ den Rest der Formel II und R² -NH₂, NH-C(O)-CH₂-NH-Schutzgruppe, -OH oder -NH-C(O)-CH₂-Halogen darstellt und anschließend
b) eine nach Verfahren a) hergestellte Verbindung, worin R⁷ -OH bedeutet, mit einem ungesättigten Alkylhalogenid, worin der Alkylteil 3, 4 oder 5 Kohlenstoffatome hat, zu einer entsprechenden Verbindung der Formel I umsetzt, oder
c) eine nach Verfahren a) hergestellt Verbindung, worin R⁷ -NH₂ bedeutet, mit einem Carbonsäurehalogenid wie Bromacetylbromid zu einer Verbindung der Formel I umsetzt, worin R² den Rest der Formel V darstellt, R³ Halogen und R⁴ Wasserstoffatom bedeuten, oder
d) ein aromatisches Diamin wie p-Phenylendiamin mit einer an der Aminogruppe geschützten Aminosäure zu einer Verbindung der Formel VII umsetzt, worin R⁷ einen Rest der Formel V darstellt, R³ -NH₂ und R⁴ geschützte Aminosäure bedeuten und anschließend wie im Verfahren a) zu einer entsprechenden Verbindung der Formel I umsetzt, oder
e) die Schutzgruppe in einer nach Verfahren a) oder d) hergestellten Verbindung der Formel I entfernt, oder
f) eine nach Verfahren a) - e) hergestellte Verbindung der Formel I, wobei R¹ für den Rest der Formel II steht, in eine Verbindung der Formel I überführt, wobei R¹ für den Rest der Formel III, steht, oder
g) die nach Verfahren a) - f) hergestellte Verbindung der Formel I entweder in freier Form isoliert oder im Falle des Vorliegens von sauren oder basischen Gruppen gegebenenfalls in physiologisch verträgliche Salze umwandelt, oder
h) eine nach Verfahren a) - g) hergestellte Verbindung der Formel I, die aufgrund ihrer chemischen Struktur in diastereoisomerer oder enantiomerer Form auftritt durch Chromatographie an einem gegebenenfalls chiralen Trägermaterial in die reinen Stereoisomeren auftrennt oder eine racemische Verbindung der Formel I, die zur Salzbildung befähigt ist, durch fraktionierte Kristallisation der mit einer optisch aktiven Base oder Säure als Hilfsstoff gebildeten diastereomeren Salze auftrennt oder eine racemische Verbindung der Formel I, die eine basische Gruppe wie eine Aminogruppe enthält, mit optisch aktiven Säuren wie (+)-Campher-10-sulfonsäure, D- und L- Weinsäure, D-und L-Milchsäure oder (+) und (-)-Mandelsäure in die reinen Enantiomeren überführt.

6. Arzneimittel, enthaltend eine Verbindung der Formel I gemäß einem oder mehreren der Ansprüche 1 bis 4 und einen pharmazeutisch verträglichen Träger.

7. Verwendung der Verbindung der Formel I gemäß einem oder mehreren der Ansprüche 1 bis 4 zur Herstellung eines Arzneimittels zur Behandlung von Krebserkrankungen, Entzündungen oder Autoimmunerkrankungen.

8. Verbindung der Formel IV 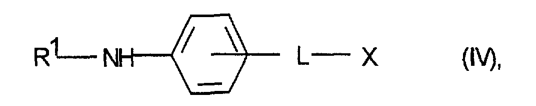 wobei
R¹ für den Rest der Formel II oder III 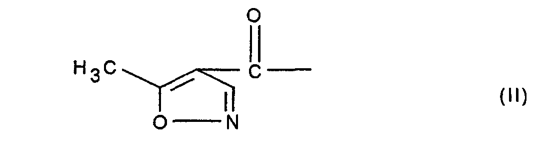 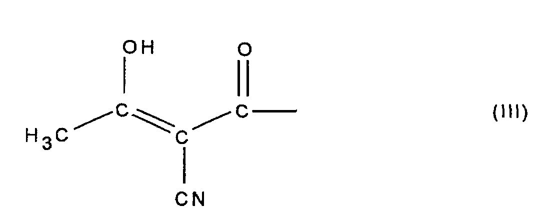
steht
L für ein Brückenglied aus der Gruppe
a) -NH-C(O)CH(R⁴)(R³),
worin
R³
a) kovalente Bindung oder
b) -NH- bedeutet und
R⁴
a) Wasserstoffatom oder
b) Rest einer Aminosäure bedeutet, oder
b) -NH-(CH₂)ₘ-S-O-, worin m die ganze Zahl von 1 bis 12 ist,
c) -NH-(CH₂)ₘ-S-NR⁵-, worin R⁵ Wasserstoffatom bedeutet und m wie oben definiert ist,
d) -NH-(CH₂)ₘ-S-O-(CH₂)ₙ)-CH₂-, worin n die ganze Zahl 1, 2 oder 3 bedeutet und m wie oben definiert ist, oder
e) -NH-(CH₂)ₘ-S-NH-C(O)-CH(R⁴)(R³), wobei R³ und R⁴ wie unter a) definiert sind und m wie oben definiert ist, bedeutet, und
X für synthetische oder natürliche Polymere aus der Gruppe Polystyrol, Polypropylen, Polyvinylchlorid, Latex, Polysaccharide, Sepharose, Proteine, Lipide, Silikate oder Nukleinsäuren steht oder für einen Festkörper steht, der ein Röhrchen, Kugel oder Mikrotiterplatte ist.

9. Verwendung der Verbindung der Formel IV' 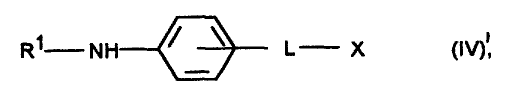 wobei
R¹ für den Rest der Formel II oder III wie definiert in Anspruch 1 steht,
L für ein Brückenglied aus der Gruppe
a) -O-,
b) -NR⁵-, worin R⁵ Wasserstoffatom bedeutet,
c) -O-(CH₂)ₙ)-CH₂-, worin n die ganze Zahl 1, 2 oder 3 bedeutet,
d) -NH-C(O)-CH(R⁴)(R³),
worin
R³
a) kovalente Bindung oder
b) -NH- bedeutet und
R⁴
a) Wasserstoffatom oder
b) Rest einer Aminosäure bedeutet, oder
e) ein Brückenglied L, definiert wie unter a) bis d), das über einen Spacer verfügt, worin der Spacer für einen Rest aus der Gruppe -NH-(CH₂)ₘ-S-, worin m die ganze Zahl von 1 bis 12 ist, steht und
X für synthetische oder natürliche Polymere aus der Gruppe Polystyrol, Polypropylen, Polyvinylchlorid, Latex, Polysaccharide, Sepharose, Proteine, Lipide, Silikate oder Nukleinsäuren steht oder für einen Festkörper steht, der ein Röhrchen, Kugel oder Mikrotiterplatte ist,
zur Modifikation von Mikrotiterplatten oder zur Herstellung von Chromatographiematerial.

10. Verwendung gemäß Anspruch 9 zur Herstellung von Affinitätschromatographiematerial.

## Claims

1. A compound of the formula I 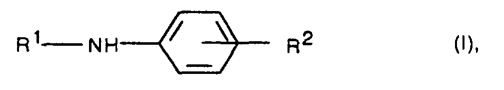 and/or a physiologically tolerable salt of the compound of the formula I and/or an optionally stereoisomeric form of the compound of the formula I,
where
R¹ is the radical of the formula II or III 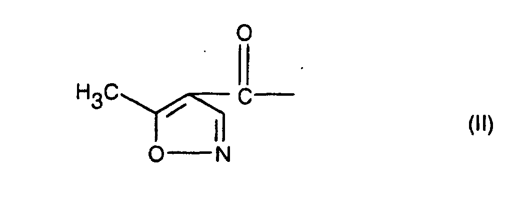 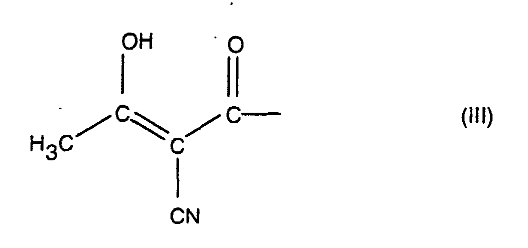
and
R² is
a) -O-(CH₂)ₙ-CH=CH₂, in which n is the integer 1, 2 or 3, or
b) the radical of the formula V 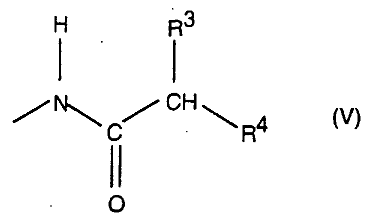 in which
R³ is
1) halogen or
2) MH₂ and
R⁴ is
1) a hydrogen atom or
2) a radical of an amino acid.

2. A compound of the formula I as claimed in claim 1, wherein
R¹ is the radical of the formula II or III and
R² is
a) -O-CH₂-CH=CH₂ or
b) -NH-C(O)-CH(R³)(R⁴),
in which R³ is bromine, -NH₂ or chlorine and R⁴ is a hydrogen atom.

3. A compound of the formula I as claimed in claim 1 or 2, wherein the radical R² in formula I is in the para-position relative to the "NH" group on the phenyl ring.

4. A compound of the formula I as claimed in claim 1, such as
2-cyano-3-hydroxybut-2-enecarboxylic acid (4-allyloxyphenyl)amide,
2-cyano-3-hydroxybut-2-enecarboxylic acid (4-(2-aminoacetylamino)phenyl)amide trifluoroacetate,
5-methylisoxazole-4-carboxylic acid (4-(2-aminoacetylamino)-phenyl)amide hydrochloride,
2-cyano-3-hydroxybut-2-enecarboxylic acid (4-(2-bromoacetylamino)phenyl)amide or
5-methylisoxazole-4-carboxylic acid (4-(2-bromoacetylamino)-phenyl)amide.

5. A process for the preparation of the compound of the formula I as claimed in one or more of claims 1 to 4, which comprises
a) reacting a compound of the formula VI 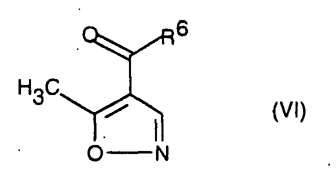 where R⁶ is the radical OH, Cl or Br, with a compound of the formula VII 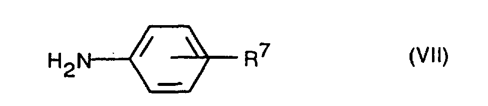 where R⁷ is
1) -NH₂,
2) an -NH-C(O)-CH₂-NH-protective group, in which protective group is an amine protective group, in particular Boc,
3) -NH-C(O)-CH₂-halogen or
4) -OH
to give a compound of the formula I in which R¹ is the radical of the formula II and R² is -NH₂, NH-C(O)-CH₂-NH-protective group, -OH or -NH-C(O)-CH₂-halogen, and then
b) reacting a compound prepared by process a), in which R⁷ is -OH, with an unsaturated alkyl halide in which the alkyl moiety has 3, 4 or 5 carbon atoms to give a corresponding compound of the formula I, or
c) reacting a compound prepared by process a), in which R⁷ is -NH₂, with a carboxylic acid halide such as bromoacetyl bromide to give a compound of the formula I in which R² is the radical of the formula V, R³ is halogen and R⁴ is a hydrogen atom, or
d) reacting an aromatic diamine such as p-phenylenediamine with an amino acid protected on the amino group to give a compound of the formula VII in which R⁷ is a radical of the formula V, R³ is -NH₂ and R⁴ is a protected amino acid and then reacting as in process a) to give a corresponding compound of the formula I, or
e) removing the protective group in a compound of the formula I prepared by process a) or d), or
f) converting a compound of the formula I prepared by processes a) - e), where R¹ is the radical of the formula II, into a compound of the formula I where R¹ is the radical of the formula III, or
g) either isolating the compound of the formula I prepared by processes a) - f) in free form or, in the case of the presence of acidic or basic groups, optionally converting it into physiologically tolerable salts, or
h) separating a compound of the formula I prepared by processes a) - g), which on account of its chemical structure occurs in diastereoisomeric or enantiomeric form, into the pure stereoisomers by chromatography on an optionally chiral support material or separating a racemic compound of the formula I, which is capable of salt formation, by fractional crystallization of the diastereomeric salts formed using an optically active base or acid as auxiliary or converting a racemic compound of the formula I which contains a basic group such as an amino group into the pure enantiomers using optically active acids such as (+)-camphor-10-sulfonic acid, D- and L-tartaric acid, D- and L-lactic acid or (+)- and (-)-mandelic acid.

6. A pharmaceutical comprising a compound of the formula I as claimed in one or more of claims 1 to 4 and a pharmaceutically tolerable excipient.

7. The use of the compound of the formula I as claimed in one or more of claims 1 to 4 for the production of a pharmaceutical for the treatment of carcinomatous diseases, inflammations or autoimmune diseases.

8. A compound of the formula IV 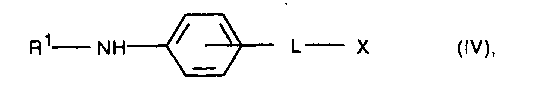 where
R¹ is the radical of the formula II or III 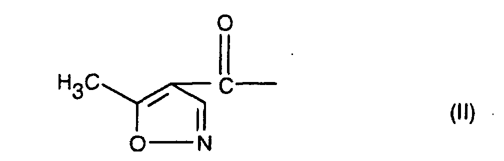 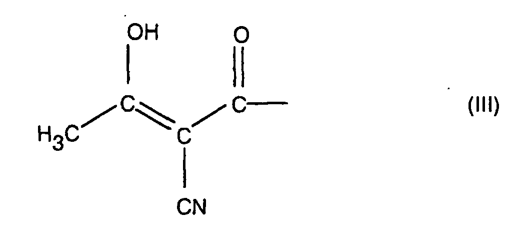
L is a bridging member from the group
a) -NH-C(O)-CH(R⁴)(R³),
where
R³ is
a) a covalent bond or
b) -NH-, and
R⁴ is
a) a hydrogen atom or
b) the radical of an amino acid, or
b) -NH-(CH₂)ₘ-S-O-, where m is an integer from 1 to 12,
c) -NH-(CH₂)ₘ-S-NR⁵-, where R⁵ is a hydrogen atom and m is as defined above,
d) -NH-(CH₂)ₘ-S-O-(CH₂)ₙ)CH₂-, where n is the integer 1, 2 or 3 and m is as defined above, or
e) -NH-(CH₂)ₘ-S-NH-C(O)-CH(R⁴)R³), where R³ and R⁴ are each as defined under a) and m is as defined above, and
X represents synthetic or natural polymers from the group consisting of polystyrene, polypropylene, polyvinyl chloride, latex, polysaccharides, Sepharose, proteins, lipids, silicates or nucleic acids or represents a solid which is a tube, a bead or a microtiter plate.

9. The use of the compound of the formula IV', 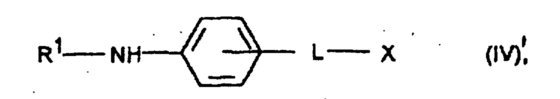 where
R¹ is the radical of the formula II or III as defined in claim 1,
L is a bridging member from the group
a) -O-,
b) -NR⁵-, in which R⁵ is a hydrogen atom,
c) -O-(CH₂)ₙ)-CH₂-, in which n is the integer 1, 2 or 3,
d) -NH-C(O)-CH(R⁴)(R³),
in which
R³ is
a) a covalent bond or
b) -NH-, and
R⁴ is
a) a hydrogen atom or
b) a radical of an amino acid, or
e) a bridging member L, defined as under a) to d), which has a spacer, in which the spacer is a radical of the group -NH-(CH₂)ₘ-S-, in which m is an integer from 1 to 12, and
X represents synthetic or natural polymers from the group consisting of polystyrene, polypropylene, polyvinyl chloride, latex, polysaccharides, Sepharose, proteins, lipids, silicates or nucleic acids or represents a solid which is a tube, a bead or a microtiter plate,
for the modification of microtiter plates or for the preparation of chromatography material.

10. The use as claimed in claim 9 for the preparation of affinity chromatography material.

## Revendications

1. Composé de formule I 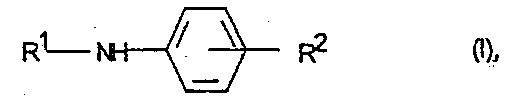 et/ou un sel physiologiquement compatible du composé de formule I et/ou éventuellement une forme stéréoisomère du composé de formule I,
où
R¹ représente la reste de formules II ou III, 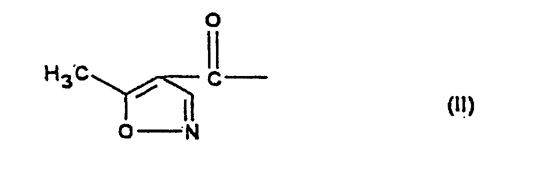 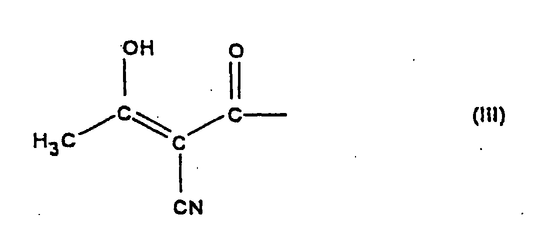
et
R² représente
a) un groupe -O- (CH₂)ₙ-CH=CH₂, où n est un entier 1, 2 ou 3, ou
b) le reste de formule V 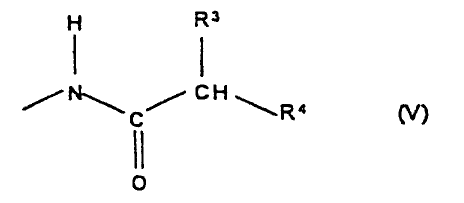 où
R³ représente
1) un atome d'halogène ou
2) un groupe NH₂ et
R⁴ représente
1) un atome d'hydrogène ou
2) le reste d'un acide aminé.

2. Composé de formule I selon la revendication 1, **caractérisé en ce que**
R¹ représente le reste de formules II ou III et
R ² représente
a) un groupe -O-CH₂-CH=CH₂ ou
b) un groupe -NH-C(O)-CH(R³)(R⁴) où
R³ représente un atome de brome, un groupe -NH₂ ou un atome de chlore et
R⁴ représente un atome d'hydrogène.

3. Composé de formule I selon la revendication 1 ou 2 **caractérisé en ce que** le reste R² dans la formule I sur le cycle phénylique se trouve en position para par rapport au groupe "NH".

4. Composé de formule I selon la revendication 1 comme
le (4-allyloxy-phényl)-amide d'acide 2-cyano-3-hydroxy-but-2-ène-carboxylique,
le trifluoroacétate de (4-(2-amino-acétylamino)-phényl)-amide d'acide 2-cyano-3-hydroxy-but-2-ène-carboxylique,
le chlorhydrate de (4-(2-amino-acétylamino)-phényl)-amide d'acide 5-méthyl-isoxazol-4-carboxylique,
le (4-(2-bromo-acétylamino)-phényl)-amide d'acide 2-cyano-3-hydroxy-but-2-ène-carboxylique ou
le (4-(2-bromacétylamino)-phényl)-amide d'acide 5-méthyl-isoxazol-4-carboxylique.

5. Procédé pour la préparation du composé de formule I selon une ou plusieurs des revendications 1 à 4, **caractérisé en ce que** l'on
a) transforme un composé de formule VI 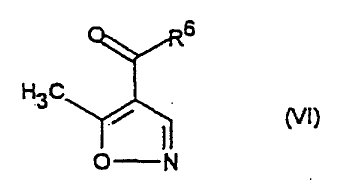 où R⁶ représente les restes OH, Cl ou Br avec un composé de formule VII 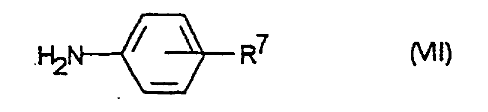 où R⁷ représente
1) un groupe -NH₂,
2) un groupe -NH-C(O)-CH₂-NH-(groupe protecteur), où le groupe protecteur représente un groupe protecteur aminé, en particulier un groupe Boc,
3) un groupé -NH-C(O)-CH₂-halogène ou
4) un groupe -OH
en un composé de formule I, où R¹ représente le reste de formule II et R² les groupes -NH₂, -NH-C(O)-CH₂-NH-(groupe protecteur), -OH ou -NH-C(O)-CH₂-halogène et ensuite
b) on transforme un composé préparé selon le procédé a), où R⁷ représente un groupe -OH, avec un alkylhalogénure insaturé, où le fragment alkyle présente 3, 4 ou 5 atomes de carbone, en un composé correspondant de formule I, ou
c) on transforme un composé préparé selon le procédé a), où R⁷ représente un groupe -NH₂, avec un halogénure d'acide carboxylique, comme le bromure de bromacétyle, en un composé de formule I, où R² représente le reste de formule V, R³ un atome d'halogène et R⁴ un atome d'hydrogène, ou
d) on transforme une diamine aromatique, comme la p-phénylèndiamine, avec un acide aminé protégé sur le groupe amino en un composé de formule VII, dans lequel R⁷ représente un reste de formule V, R³ un groupe -NH₂ et R⁴ un acide aminé protégé et ensuite, comme dans le procédé a), on le transforme en un composé de formule I correspondant, ou
e) on élimine le groupe protecteur dans un composé de formule I préparé selon le procédé a) ou d), ou
f) on convertit un composé de formule I préparé selon les procédés a) à e), dans lequel R¹ représente le reste de formule II, en un composé de formule I, où R¹ représente le reste de formule III, ou
g) soit on isole sous forme libre le composé de formule I préparé selon les procédés a) à f), soit, dans des cas de présence de groupes acides ou basiques, on le transforme éventuellement en sels physiologiquement compatibles, ou
h) on sépare un composé de formule I préparé selon les procédés a) à g) qui, en raison de sa structure chimique, apparaît sous forme diastéréoisomère ou énantiomère, par chromatographie sur une matière support éventuellement chirale en les stéréoisomères purs ou on sépare un composé de formule I racémique, qui est capable de former un sel, par cristallisation fractionnée des sels diastéréomères formés avec une base ou un acide optiquement actif en tant qu'adjuvant ou on transforme un composé de formule I racémique, qui comporte un groupe basique tel qu'un groupe amino, avec des acides optiquement actifs comme l'acide (+)-camphor-10-sulfonique les acides D-et L-tartriques, les acides D- et L-lactiques ou les acides phényl-glycoliques (+) et (-) en énantiomères purs.

6. Médicament, contenant un composé de formule I selon une ou plusieurs des revendications 1 à 4 et un véhicule pharmaceutiquement compatible.

7. Utilisation du composé de formule I selon une ou plusieurs des revendications 1 à 4 pour la préparation d'un médicament pour le traitement de maladies cancéreuses, d'inflammations ou de maladies auto-immunes.

8. Composé de formule IV 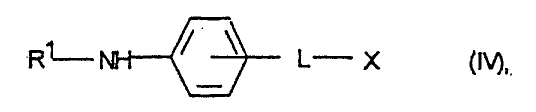 où
R¹ représente le reste de formule II ou III 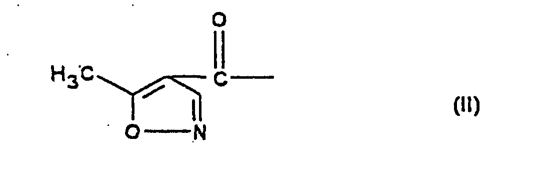 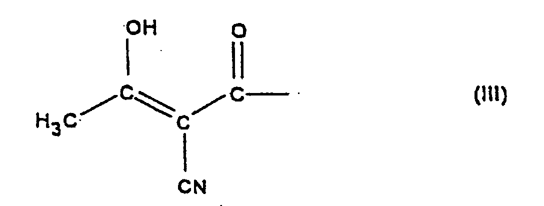
L représente un élément de pontage pris dans le groupe
a) -NH-C(O)-CH(R⁴) (R³),
où
R³ représente
a) une liaison covalente ou
b) un groupe -NH et
R⁴ représente
a) un atome d'hydrogène ou
b) le reste d'un acide aminé, ou
b) -NH-(CH₂)ₘ-S-O-, où m est un entier de 1 à 12,
c) -NH-(CH₂)ₘ-S-NR₅-, où R⁵ représente un atome d'hydrogène et m est défini comme ci-dessus,
d) NH-(CH₂)ₘ-S-O-(CH₂)ₙ-CH₂-, dans lequel n vaut le nombre entier 1, 2 ou 3 et m est défini comme ci-dessus, ou
e) -NH-(CH₂)ₘ-S-NH-C(O)-CH(R⁴) (R³), où R³ et R⁴ sont définis comme en a) et m est défini comme ci-dessus, et
X représente des polymères synthétiques ou naturels de l'ensemble comprenant le polystyrène, le polypropylène, le poly(chlorure de vinyle), le latex, des polysaccharides, la Sépharose, des protéines, des lipides, des silicates ou des acides nucléiques ou un corps solide, qui est un tube, une bille ou une plaque de microtitration.

9. Utilisation du composé de formule IV' 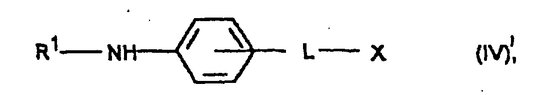 où
R¹ représente le reste de formule II ou III comme défini dans la revendication 1,
L représente un élément de pontage pris dans le groupe
a) -O-,
b) -NR⁵-, dans lequel R⁵ représente un atome d'hydrogène,
c) -O-(CH₂)ₙ-CH₂-, dans lequel n est un entier 1, 2 ou 3,
d) -NH-C(O)-CH(R⁴) (R³),
où
R³ représente
a) une liaison covalente ou
b) un groupe -NH et
R⁴ représente
a) un atome d'hydrogène ou
b) le reste d'un acide aminé, ou
e) un élément de pontage L, défini comme aux points a) à d), qui dispose d'un espaceur, dans lequel l'espaceur représente un reste pris dans le groupe -NH-(CH₂)ₘ-S-, dans lequel m est un entier de 1 à 12, et
X représente des polymères synthétiques ou naturels pris dans le groupe comprenant le polystyrène, le polypropylène, le poly(chlorure de vinyle), le latex, des polysaccharides, la Sépharose, des protéines, des lipides, des silicates ou des acides nucléiques ou un corps solide, qui est un tube, une bille ou une plaque de microtitration,
pour la modification de plaques de microtitration ou pour la préparation de matière de chromatographie.

10. Utilisation selon la revendication 9 pour la préparation d'une matière de chromatographie d'affinité.
